# EUROPÄISCHE PATENTANMELDUNG

(11) **EP 2 966 432 A1**
(43) Veröffentlichungstag der Anmeldung: **13.01.2016**
(21) Anmeldenummer: 15175838.0
(22) Anmeldetag: 08.07.2015
(51) Int. Cl.: G01N 15/08, A61B 5/00

(54) **VORRICHTUNG ZUR MESSUNG EINER DURCH DIFFUSION DURCH EIN PROBENMATERIAL ABGEGEBENEN MENGE EINES STOFFES**

(30) Priorität: 08.07.2014 DE 202014005525 U
(71) Anmelder: Courage + Khazaka electronic GmbH, 50829 Köln (DE)
(72) Erfinder: Khazaka, Gabriel, 50859 Köln (DE)
(74) Vertreter: von Kreisler Selting Werner - Partnerschaft von Patentanwälten und Rechtsanwälten mbB

(57) **Zusammenfassung**

Bei einer Vorrichtung (20) zur Messung einer durch Diffusion durch ein Probenmaterial (6) abgegebenen Menge eines Stoffes, mit einem über dem Probenmaterial (6) platzierbaren offenen Messkopf (28), wobei der Messkopf (28) mindestens zwei Öffnungen (24, 26) aufweist, wobei eine der mindestens zwei Öffnungen (26) auf dem zu untersuchenden Probenmaterial (6) platzierbar ist, wobei in dem Messkopf (28) mindestens eine erste und eine zweite Sensoreinrichtungen (30, 32) angeordnet sind, wobei die erste und die zweite Sensoreinrichtung (30, 32) beim Messen in unterschiedlichen Abständen von dem zu untersuchenden Probenmaterial (6) angeordnet sind, ist vorgesehen, dass der Messkopf (28) auf der dem Probenmaterial (6) zugewandten Seite eine Anschlusseinrichtung (31) aufweist, die an ein Franz-Zellen Unterteil (2) angepasst ist, so dass die Vorrichtung (20) an ein Franz-Zellen Unterteil (2) anschließbar ist.

## Beschreibung

Die Erfindung betrifft eine Vorrichtung zur Messung eine durch Diffusion durch ein Probenmaterial abgegebenen Menge eines Stoffes nach dem Oberbegriff des Anspruchs 1.

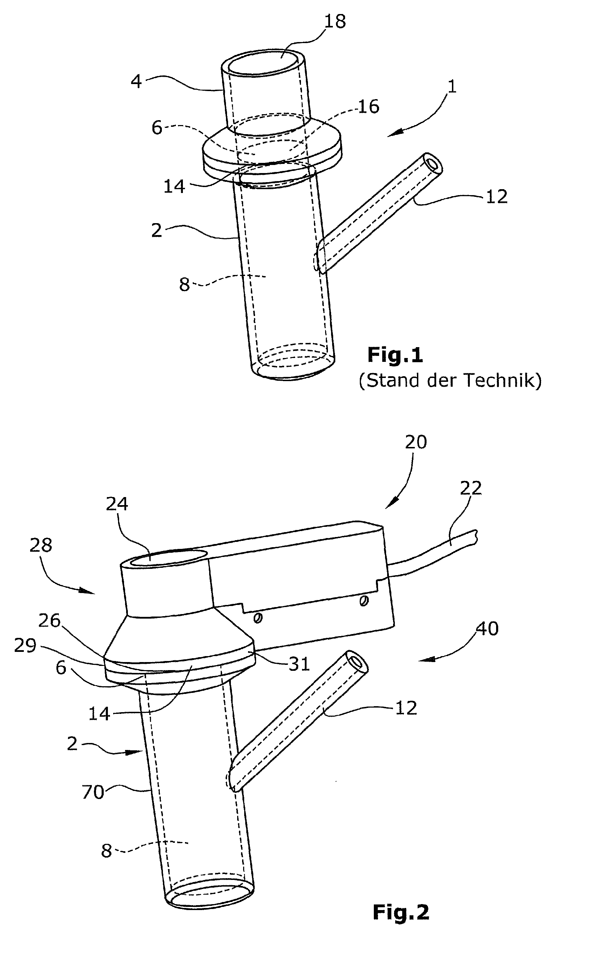

Es sind gemäß dem Stand der Technik Franz-Zellen bekannt, die eine einfache und sehr häufig verwendete Methode darstellen, um Tests für kosmetische und pharmazeutische Produkte durchzuführen. Eine Franz-Zelle gemäß dem Stand der Technik ist in Fig. 1 dargestellt. Die Franz-Zelle 1 weist ein Franz-Zellen Unterteil 2 und ein Franz-Zellen Oberteil 4 auf. Das Franz-Zellen Unterteil besteht vorzugsweise aus Glas. Zwischen dem Unterteil 2 und dem Oberteil 4 ist ein Probenmaterial 6 eingespannt. Das Unterteil 2 weist eine Kammer 8 auf, in die eine Flüssigkeit durch den seitlichen Rohrfortsatz 12 eingeführt werden kann. Das Unterteil 2 weist an der dem Oberteil 4 zugewandten Seite eine Öffnung 14 auf. Das Oberteil 4 weist auf der dem Unterteil 2 zugewandten Seite eine Öffnung 16 und an der dem Unterteil 2 abgewandten Seite eine Öffnung 18 auf. Durch Erhitzen der gesamten Franz-Zelle entsteht im Unterteil ein Druck, der die Flüssigkeit, die im Unterteil 2 angeordnet ist, durch das Probenmaterial drückt. Mit Hilfe der Franz-Zelle kann insbesondere die Wasserdurchlässigkeit des Probenmaterials gemessen werden. Dafür wird eine Sonde auf den Aufbau der Franz-Zelle, d. h. auf das Oberteil 4 aufgesetzt. Es besteht jedoch das Problem, dass eine solche Messung nicht mit einer Messung, die direkt auf einer menschlichen Haut durchgeführt wird, vergleichbar ist.

Der Erfindung liegt daher die Aufgabe zugrunde, eine Vorrichtung zu schaffen, bei der die Messung einer durch Diffusion durch ein Probenmaterial abgegebenen Menge eines Stoffs verbessert wird.

Zur Lösung dieser Aufgabe dienen die Merkmale des Anspruchs 1.

Die Erfindung sieht in vorteilhafter Weise vor, dass die Vorrichtung zur Messung einer durch Diffusion durch ein Probenmaterial abgegebenen Menge eines Stoffs, mit
- einem über dem Probenmaterial platzierbaren offenen Messkopf, wobei der Messkopf mindestens eine erste und eine zweite Öffnung aufweist, wobei die erste Öffnung auf dem zu untersuchenden Probenmaterial platzierbar ist,
- wobei in dem Messkopf mindestens eine erste und eine zweite Sensoreinrichtung angeordnet sind, wobei die erste und die zweite Sensoreinrichtung beim Messen in unterschiedlichen Abständen von dem zu untersuchenden Probenmaterial angeordnet sind,
   vorgesehen ist, dass
- der Messkopf auf der dem Probenmaterial zugewandten Seite eine Anschlusseinrichtung aufweist, die an ein Franz-Zellen Unterteil angepasst ist, so dass die Vorrichtung an dem Franz-Zellen Unterteil anschließbar ist.

Die mindestens eine erste und die mindestens eine zweite Sensoreinrichtung können Temperatur und Luftfeuchtigkeit messen.

Es kann eine dritte Sensoreinrichtung vorgesehen sein, die die Temperatur der Oberfläche des Probenmaterials misst. Diese dritte Sensoreinrichtung kann vorzugsweise nahe der ersten Öffnung angeordnet sein, so dass die dritte Sensoreinrichtung direkt auf oder nahe des zu untersuchenden Probenmaterials angeordnet sein kann.

Mit Hilfe dieses mittels der dritten Sensoreinrichtung gemessenen zusätzlichen Messwertes kann die Durchlässigkeit des Probenmaterials noch genauer bestimmt werden.

Zwischen den beiden Öffnungen kann ein Messraum angeordnet sein, der vorzugsweise zylinderförmig ausgebildet ist und in dem die Sensoreneinrichtungen angeordnet sind. Der Messraum kann auch aufgrund der sich gegenüberliegenden Öffnungen Kamin genannt werden.

Es kann ein Trägereinrichtung vorgesehen sein, die zumindest teilweise in dem Messraum hineinragt, wobei die mindestens eine erste und die mindestens eine zweite Sensoreinrichtung auf dem in den Messraum hineinragenden Teil des Trägereinrichtung angeordnet sind. Die Trägereinrichtung kann eine Leiterplatte sein.

Auch die mindestens eine dritte Sensoreinrichtung kann auf einem in dem Messraum hineinragenden Teil der Trägereinrichtung angeordnet sein.

Es kann mittels eines Adapters die Breite des Messraums, bzw. die Breite des Kamins, einstellbar sein.

Die erste und die zweite Sensoreinrichtung können in einem vordefinierten Abstand zu der Oberfläche des Probenmaterials angeordnet sein.

Die zweite Sensoreinrichtung, die näher als die erste Sensoreinrichtung an der Oberfläche des Probenmaterials angeordnet ist, kann 3 - 5 mm, insbesondere ca. 4 mm, von der Oberfläche des Probenmaterials entfernt angeordnet sein.

Es können Anschlussleitungen zum Übertragen der von der Vorrichtung gemessenen Messwerte oder eine Funkeinrichtung zum Übertragen der gemessenen Messwerte an eine Auswerteeinrichtung und/oder Anzeigeeinrichtung vorgesehen sein.

Es kann ferner eine Einrichtung mit einem Franz-Zellen-Unterteil, einer Vorrichtung nach einem der Ansprüche 1 bis 9 und einem zwischem dem Franz-Zellen Unterteil und der Vorrichtung nach einem der Ansprüche 1 bis 9 angeordneten Probenmaterial vorgesehen sein, wobei das Franz-Zellen Unterteil ein Gehäuse aufweist, das im Inneren eine Kammer zur Aufnahme einer Flüssigkeit aufweist, wobei die Kammer an dem Ende, das der Vorrichtung nach einem der Ansprüche 1 - 9 zugewandt ist, offen ist.

Ferner kann ein System mit mehreren Einrichtungen nach Anspruch 10 und einer Auswerteeinrichtung zum Auswerten der von den mehreren Einrichtungen gemessenen Messwerte vorgesehen sein. Es können bis zu 20 Einrichtungen nach Anspruch 10 mit der Auswerteeinrichtung verbunden sein.

Im Folgenden wird unter Bezugnahme auf die Zeichnungen ein Ausführungsbeispiel der Erfindung näher erläutert.

Es zeigen schematisch:
- Fig. 1: eine Franz-Zelle gemäß dem Stand der Technik,
- Fig. 2: eine erfindungsgemäße Vorrichtung zur Messung einer durch Diffusion durch ein Probenmaterial abgegebenen Menge eines Stoffs auf einem Franz-Zellen Unterteil,
- Fig. 3: die Vorrichtung zur Messung einer durch Diffusion durch ein Probenmaterial abgegebenen Menge eines Stoffs aus Fig. 2 in der Draufsicht,
- Fig. 4: zeigt die Vorrichtung aus Fig. 3 in einer geschnittenen Seitenansicht,
- Fig. 5: zeigt ein alternatives Ausführungsbeispiel zu Fig. 4.
- Fig. 6: zeigt ein System mit Einrichtungen aus Fig. 2 sowie eine Auswerteeinheit.

Fig. 2 zeigt eine Vorrichtung 20 zur Messung einer durch Diffusion durch ein Probenmaterial 6 abgegebenen Menge eines Stoffs gemäß der vorliegenden Erfindung. Die Vorrichtung 20 weist einen offenen Messkopf 28 auf. Der Messkopf 28 weist mindestens eine erste und eine zweite Öffnung 24, 26 auf, wobei eine der mindestens zwei Öffnungen 24, 26 dem das zu untersuchenden Probenmaterial 6 platzierbar ist. Im dargestellten Ausführungsbeispiel ist die erste Öffnung 26 auf dem zu untersuchenden Probenmaterial 6 platziert.

Der Messkopf 28 weist auf der dem Probenmaterial 6 zugewandten Seite 29 eine Anschlusseinrichtung 31 auf, die an ein Franz-Zellen Unterteil 2 angepasst ist, so dass die Vorrichtung 20 an das Franz-Zellen Unterteil 2 anschließbar ist. Das Franz-Zellen Unterteil 2 gemäß der vorliegenden Erfindung ist ein Franz-Zellen Unterteil, wie es in Fig. 1 beschrieben worden ist und aus dem Stand der Technik bekannt ist. Dieses Franz-Zellen-Unterteil 2 weist eine Kammer 8 auf, die eine Öffnung 14 aufweist. Das Franz-Zellen Unterteil 2 besteht vorzugsweise aus Glas. Die Öffnung 14 ist auf der der Vorrichtung 20 zugewandten Seite des Franz-Zellen Unterteils 2 angeordnet. Zwischen dem Franz-Zellen Unterteil 2 und der Vorrichtung 20 ist ein Probenmaterial 6 eingespannt. Das Probenmaterial 6 ist vorzugsweise eine Membran. Das Franz-Zellen-Unterteil 2 weist ferner einen Rohrstutzen 12 auf, durch den Flüssigkeit in die Kammer des Franz-Zellen-Unterteils 2 eingeleitet werden kann.

Mittels der Anschlusseinrichtung 31 kann die Vorrichtung 20 mit dem Franz-Zellen Unterteil 2 verbunden werden. Es können auch zusätzliche Verbindungsmittel vorgesehen sein, wie beispielsweise Klammern, um das Franz-Zellen Unterteil 2 und die Vorrichtung 20 miteinander zu verbinden. Die Anschlusseinrichtung 30 ist in jedem Fall derart geformt, dass sie der Form der Oberseite 7 des Franz-Zellen-Unterteils 20 angepasst ist, so dass diese aufeinander passen.

Die Öffnung 14 des Franz-Zellen-Unterteils 2 ist an die Größe der Öffnung 26 der Vorrichtung 20 angepasst.

Die Vorrichtung 20, das Franz-Zellen-Unterteil 2 und das dazwischen eingespannte Probenmaterial bilden eine Einrichtung 40 nach Anspruch 11.

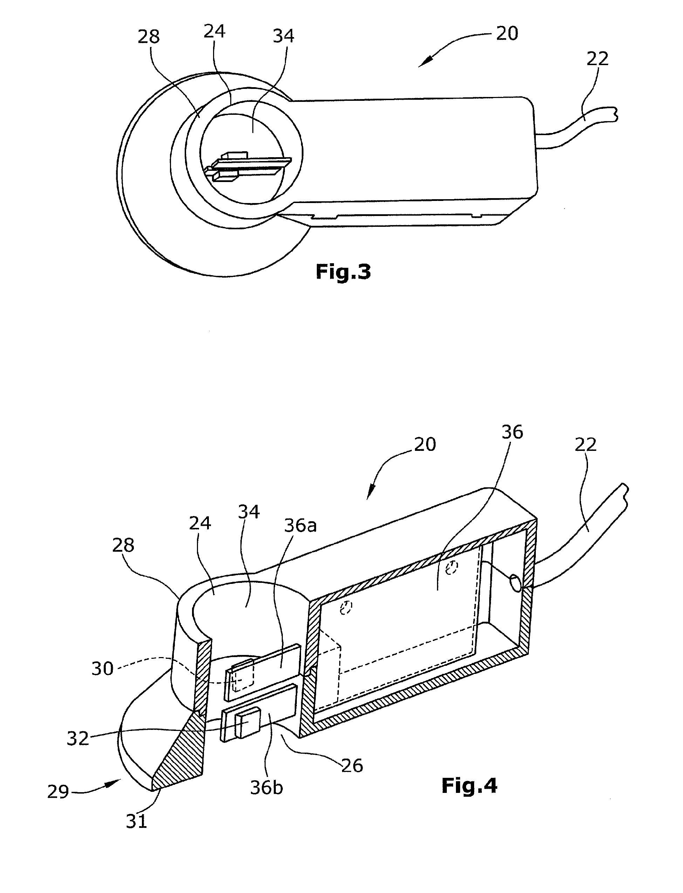

In Fign. 3 und 4 ist die Vorrichtung 20 näher dargestellt. Wie den Fign. 3 und 4 entnommen werden kann, weist der Messkopf 28 einen Messraum 34 auf. Der Messraum 34 erstreckt sich von der ersten Öffnung 24 der Vorrichtung 20 zu der zweiten Öffnung 26 der Vorrichtung 20. Die Vorrichtung 20 weist mindestens eine erste und eine zweite Sensoreinrichtung 30, 32 auf. Die erste und die zweite Sensoreinrichtung 30, 32 sind vorzugsweise in dem Messraum 34 des Messkoffers 28 angeordnet. Der Messraum 34 ist vorzugsweise kaminförmig, insbesondere zylinderkreisförmig ausgebildet. Die erste und die zweite Sensoreinrichtung 30,32 können die Temperatur und die relative Luftfeuchtigkeit messen.

Das Messprinzip beruht vorzugsweise auf den Fick'schen Diffusionsgesetz. Die erste und die zweite Sensoreinrichtung 30, 32 weisen jeweils einen vordefinierten Abstand jedoch unterschiedlichen Abstand von der Oberfläche des Probenmaterials 6 aufweisen. Mittels der ersten und der zweiten Sensoreinrichtung 30, 32 wird die Temperatur und die relative Luftfeuchtigkeit gemessen. Die durch Diffusion durch das Probenmaterial 6 abgegebene Menge eines Stoffs kann vorzugsweise verdunstetes Wasser sein. Der Partialdruck des Wasserdampfes wird von der ersten und der zweiten Sensoreinrichtung 30,32 gemessen. Das Gefälle des mittels der ersten und der zweiten Sensoreinrichtung 30,32 gemessenem Partialdrucks ist zum Verdunstungsgrad direkt proportional, so dass die Wasserdurchlässigkeit des Probenmaterials 6 bestimmt werden kann.

Die erste und die zweite Sensoreinrichtung 30, 32 sind in einer Richtung, die parallel zu dem Probenmaterial 6 verläuft, vorzugsweise mittig angeordnet.

In einem nicht dargestellten alternativen Ausführungsbeispiel kann ein Adapter vorgesehen sein, mittels dem die Breite bzw. der Durchmesser des Messraums 34 eingestellt werden kann. D. h. die Breite bzw. der Durchmesser des Messraums 34 des Messkopfs 28 kann mittels des nicht dargestellten Adapters verkleinert oder vergrößert werden. Der nicht dargestellte Adapter kann beispielsweise ein Hohlzylinder sein, dessen Außenmaße an die Maße des Messraums 34 angepasst sind.

In Fig. 4 ist die Vorrichtung 20 in einer geschnittenen Ansicht dargestellt. Aus Fig. 4 geht hervor, dass die Vorrichtung 20 eine Trägereinrichtung aufweist, die vorzugsweise eine Leiterplatte 36 ist. Die erste und die zweite Sensoreinrichtung 30, 32 sind jeweils auf Teilen 36a und 36b der Leiterplatte 36, die in dem Messraum 34 hineinragen, angeordnet. Die Vorrichtung 20 kann ferner eine Anschlussleitung 22 aufweisen, die zu einer nicht dargestellten Auswerteeinheit und/oder eine Anzeigeeinheit führt, so dass die mittels der Vorrichtung 20 gemessenen Messwerte in der Auswerteeinheit verarbeitet und mit der Anzeigeeinheit angezeigt werden können.

Alternativ zur der Anschlussleitung 22 kann die Vorrichtung 20 eine Funkeinrichtung aufweisen, mittels der die Messergebnisse an die Auswerteeinheit und/oder die Anzeigeeinheit kabellos übermittelt werden können.

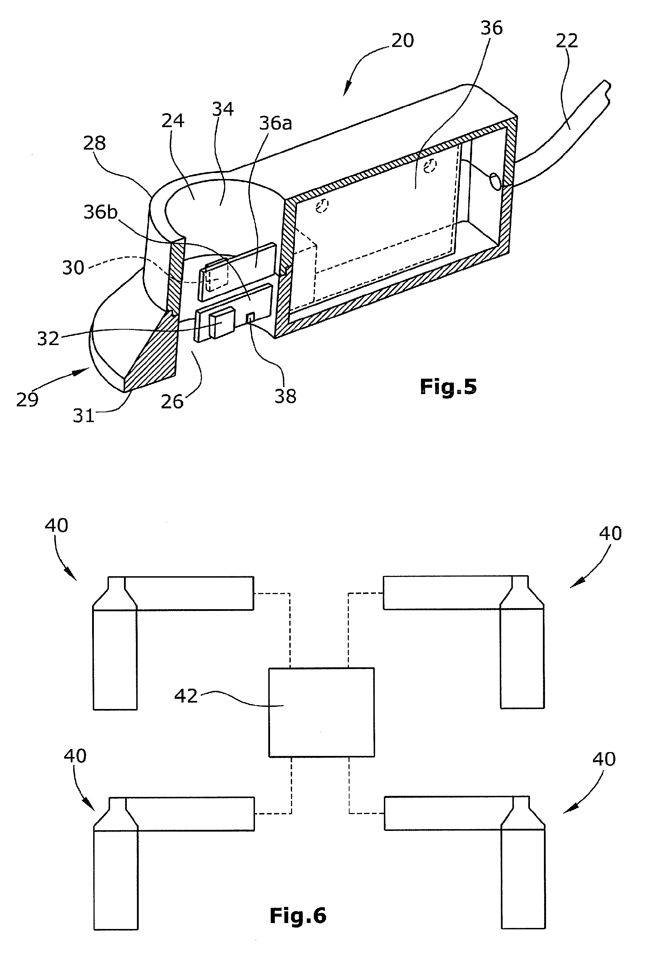

In Fig. 5 ist ein alternatives Ausführungsbeispiel zu Fig. 4 dargestellt. In Fig. 5 ist eine dritte Sensoreinrichtung 38 vorgesehen, wobei mittels dieser dritten Sensoreinrichtung die Oberflächentemperatur des Probenmaterials 6 gemessen werden kann. Dafür weist die dritte Sensoreinrichtung einen Temperaturmesssensor auf, der kontaktlos oder mit Kontakt die Temperatur des Probenmaterials 6 messen kann. Die dritte Sensoreinrichtung 38 kann vorzugsweise nahe der ersten Öffnung 26 angeordnet sein, so dass die dritte Sensoreinrichtung 38 nahe oder auf dem Probenmaterial 6 angeordnet ist.

Fig. 6 zeigt ein System mit mehreren Einrichtungen 40, die in Fig. 2 dargestellt sind, und eine Auswerteeinheit 42 zum Auswerten der von den mehreren Einrichtungen 40 gemessenen Messwerte. Es können bis zu zwanzig solcher Einrichtungen 40 mit der Auswerteeinrichtung 42 verbunden sein und die Auswerteeinheit 42 kann alle diese Einrichtungen 40 auswerten.

## Patentansprüche

1. Vorrichtung (20) zur Messung einer durch Diffusion durch ein Probenmaterial (6) abgegebenen Menge eines Stoffes, mit
- einem über dem Probenmaterial (6) platzierbaren offenen Messkopf (28), wobei der Messkopf (28) mindestens zwei Öffnungen (24, 26) aufweist, wobei eine der mindestens zwei Öffnungen (26) auf dem zu untersuchenden Probenmaterial (6) platzierbar ist,
- wobei in dem Messkopf (28) mindestens eine erste und eine zweite Sensoreinrichtungen (30, 32) angeordnet sind, wobei die erste und die zweite Sensoreinrichtung (30, 32) beim Messen in unterschiedlichen Abständen von dem zu untersuchenden Probenmaterial (6) angeordnet sind,
**dadurch gekennzeichnet,**
**dass** der Messkopf (28) auf der dem Probenmaterial (6) zugewandten Seite eine Anschlusseinrichtung (31) aufweist, die an ein Franz-Zellen Unterteil (2) angepasst ist, so dass die Vorrichtung (20) an ein Franz-Zellen Unterteil (2) anschließbar ist.

2. Vorrichtung (20) nach Anspruch 1, **dadurch gekennzeichnet, dass** die mindestens eine erste und die mindestens eine zweite Sensoreinrichtung (30, 32) Temperatur und relative Luftfeuchtigkeit messen.

3. Vorrichtung (20) nach Anspruch 1 oder 2, **dadurch gekennzeichnet, dass** eine dritte Sensoreinrichtung vorgesehen ist, die die Temperatur der Oberfläche des Probenmaterials (6) misst.

4. Vorrichtung (20) nach einem der Ansprüche 1 bis 3, **dadurch gekennzeichnet, dass** zwischen den beiden Öffnungen (24, 26) ein Messraum (34) angeordnet ist, der vorzugsweise zylinderförmig ausgebildet ist und in dem die Sensoreneinrichtungen (30, 32) angeordnet sind.

5. Vorrichtung (20) nach einem der Ansprüche 1 bis 4, **dadurch gekennzeichnet, dass** eine Trägereinrichtung vorgesehen ist, die zumindest teilweise in den Messraum (34) hineinragt, wobei die mindestens eine erste und die mindestens eine zweite Sensoreinrichtung (30, 32) auf dem in den Messraum hineinragenden Teil der Trägereinrichtung angeordnet sind.

6. Vorrichtung (20) nach einem der Ansprüche 1 bis 5, **dadurch gekennzeichnet, dass** mittels eines Adapters die Breite des Messraums (34) einstellbar ist.

7. Vorrichtung (20) nach einem der Ansprüche 1 bis 6, **dadurch gekennzeichnet, dass** die erste und die zweite Sensoreinrichtung (30, 32) in einem vordefinierten Abstand zu der Oberfläche des Probenmaterials (6) angeordnet ist.

8. Vorrichtung (20) nach Anspruch 7, **dadurch gekennzeichnet, dass** die zweite Sensoreinrichtung (32), die näher als die erste Sensoreinrichtung (30) an der Oberfläche des Probenmaterials (6) angeordnet ist, 3 - 5 mm, vorzugsweise 4mm von der Oberfläche des Probenmaterials entfernt angeordnet ist.

9. Vorrichtung (20) nach einem der Ansprüche 1 bis 8, **dadurch gekennzeichnet, dass** mindestens eine Anschlussleitung (22) zum Übertragen der von der Vorrichtung (20) gemessenen Messwerte oder eine Funkeinrichtung zum Übertragen der gemessenen Messwerte an eine Auswerteeinrichtung (42) und/oder Anzeigeeinrichtung vorgesehen sind.

10. Einrichtung (40) mit einem Franz-Zellen Unterteil (2), einer Vorrichtung (20) nach einem der Ansprüche 1 bis 9 und einem zwischen dem Franz-Zellen Unterteil (2) und der Vorrichtung (20) nach einem der Ansprüche 1 bis 9 angeordneten Probenmaterial (6), wobei das Franz-Zellen Unterteil (2) ein Gehäuse (70) aufweist, das im Innern eine Kammer (8) zur Aufnahme einer Flüssigkeit aufweist, wobei die Kammer an dem Ende, das der Vorrichtung (20) nach einem der Ansprüche 1 bis 9 zugewandt ist, offen ist.

11. System mit mehreren Einrichtungen (40) nach Anspruch 10 und einer Auswerteeinrichtung (42) zum Auswerten der von den mehreren Einrichtungen (40) gemessenen Messwerten.
